(19) Europäisches Patentamt
European Patent Office
Office européen des brevets

(11) EP 2 196 180 A2

(12) DEMANDE DE BREVET EUROPEEN

(43) Date de publication:
**16.06.2010 Bulletin 2010/24**

(21) Numéro de dépôt: **09178927.1**

(22) Date de dépôt: **11.12.2009**

(51) Int Cl.:
***A61K 8/19*** (2006.01) ***A61K 8/22*** (2006.01)
***A61K 8/27*** (2006.01) ***A61K 8/34*** (2006.01)
***A61K 8/365*** (2006.01) ***A61K 8/368*** (2006.01)
***A61K 8/44*** (2006.01) ***A61K 8/49*** (2006.01)
***A61K 8/60*** (2006.01) ***A61Q 5/10*** (2006.01)

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**
Etats d'extension désignés:
**AL BA RS**

(30) Priorité: **12.12.2008 FR 0858557**

(71) Demandeur: **L'Oréal**
**75008 Paris (FR)**

(72) Inventeurs:
- **Guerin, Frédéric**
**75010, Paris (FR)**
- **Gourlaouen, Luc**
**92600, Asnieres (FR)**

(74) Mandataire: **Fevrier, Murielle Françoise E.**
**L'Oréal**
**River Plaza - DIPI**
**25-29 Quai Aulagnier**
**92665 Asnières-sur-Seine (FR)**

# (54) Procédé de coloration capillaire à partir d'une composition comprenant au moins un orthodiphenol, un sel métallique, du péroxyde d'hydrogène et du (bi)carbonate

(57) L'invention a pour objet un procédé de coloration de fibres kératiniques par application sur lesdites fibres i) d'au moins un dérivé d'orthodiphénol, ii) d'au moins un sel métallique, iii) d'au moins du peroxyde d'hydrogène ou d'au moins un système générateur de peroxyde d'hydrogène et iv) d'au moins du (bi)carbonate.

Un autre objet de l'invention est un dispositif à plusieurs compartiments comprenant les ingrédients i), ii) et iii) et iv).

Ce procédé de coloration capillaire permet d'obtenir de meilleures colorations plus homogènes, chromatiques, tenaces et qui n'altèrent pas les propriétés cosmétiques des fibres kératiniques à partir d'extrait d'orthodiphénols notamment naturels.



Printed by Jouve, 75001 PARIS (FR)

## Description

L'invention a pour objet un procédé de coloration de fibres kératiniques par traitement desdites fibres avec i) au moins un dérivé d'orthodiphénol, ii) au moins un sel métallique, iii) au moins du peroxyde d'hydrogène ou au moins un système générateur de peroxyde d'hydrogène et iv) au moins du (bi)carbonate.

Il est connu d'obtenir des colorations dites « permanentes » avec des compositions tinctoriales contenant des précurseurs de colorant d'oxydation, appelés généralement bases d'oxydation, tels que des ortho ou para-phénylènediamines, des ortho ou para-aminophénols et des composés hétérocycliques. Ces bases d'oxydation sont des composés incolores ou faiblement colorés qui, associés à des produits oxydants, peuvent donner naissance par un processus de condensation oxydative à des composés colorés. On sait également que l'on peut faire varier les nuances obtenues en associant ces bases d'oxydation à des coupleurs ou modificateurs de coloration, ces derniers étant choisis notamment parmi les méta-diamines aromatiques, les méta-aminophénols, les méta-diphénols et certains composés hétérocycliques tels que des composés indoliques. Ce procédé de coloration d'oxydation consiste à appliquer sur les fibres kératiniques, des bases ou un mélange de bases et de coupleurs avec du peroxyde d'hydrogène ($H_2O_2$ ou eau oxygénée) à titre d'agent oxydant, à laisser diffuser, puis à rincer les fibres. Les colorations qui en résultent sont permanentes, puissantes, et résistantes aux agents extérieurs, notamment à la lumière, aux intempéries, aux lavages, à la transpiration et aux frottements,

Cependant les colorations capillaires commerciales qui les contiennent peuvent présenter des inconvénients comme le tachage, les problèmes d'odeur, de confort et de dégradation des fibres kératiniques. C'est particulièrement le cas avec les colorations d'oxydation.

Dans le domaine de la coloration, il est également connu de colorer des matières kératiniques telles que les cheveux ou la peau à partir de composés orthodiphénols en présence d'un sel métallique notamment de Mn et/ou de Zn. En particulier les demandes de brevets FR 2 814 943, FR 2 814 945, FR 2 814 946, FR 2 814 947, proposent des compositions pour la coloration de la peau ou des fibres kératiniques, comprenant un précurseur de colorant qui contient au moins un orthodiphénol, des sels et oxydes de Mn et/ou Zn, des alcalins de type hydrogénocarbonate dans un ratio particulier Mn, Zn / hydrogénocarbonate et éventuellement une enzyme. Selon ces documents il est possible d'obtenir des colorations intenses en s'affranchissant du peroxyde d'hydrogène. Cependant les colorations obtenues sont insuffisamment intenses notamment dans le cas des fibres capillaires.

Il existe un besoin de développer des procédés de colorations qui permettent d'obtenir des colorations puissantes à partir d'orthodiphénols, notamment à partir d'extrait naturel riche en orthodiphénols, tout en limitant la décoloration des fibres kératiniques. En particulier, il existe un besoin d'obtenir des colorations moins agressives pour les cheveux et dans un même temps qui résistent aux agents extérieurs (lumière, intempéries, shampooings), qui soient tenaces et homogènes tout en restant puissantes et chromatiques.

Ce but est atteint par la présente invention qui a pour objet un procédé de coloration des fibres kératiniques, dans lequel lesdites fibres sont traitées par :

i) un ou plusieurs dérivé(s) d'orthodiphénol(s),
ii) un ou plusieurs sel(s) métallique(s),
iii) du peroxyde d'hydrogène ou un ou plusieurs système(s) générateur(s) de peroxyde d'hydrogène ; et
iv) un ou plusieurs (bi)carbonate(s)

étant entendu que lorsque le procédé met en oeuvre un ou plusieurs système(s) générateur(s) de peroxyde d'hydrogène ledit procédé est réalisé en plusieurs étapes et le ou les (bi)carbonate(s) ne peuvent pas se retrouver appliqué(s) ensemble(s) sur les fibres avec le ou les sel(s) métallique(s).

Un autre objet de l'invention concerne une composition cosmétique pour la coloration des fibres kératinique comprenant :

i) un ou plusieurs dérivé(s) d'orthodiphénol(s),
ii) un ou plusieurs sel(s) métallique(s),
iii) du peroxyde d'hydrogène ou un ou plusieurs système(s) générateur(s) de peroxyde d'hydrogène ; et
iv) un ou plusieurs (bi)carbonate(s). Selon un mode réalisation particulier de l'invention la composition est une composition aqueuse.

Un autre objet de l'invention concerne un dispositif à plusieurs compartiments comprenant les ingrédients i) à iv) tels que définis précédemment.

Le procédé selon l'invention présente l'avantage de colorer les fibres kératiniques humaines, avec des résultats de colorations puissantes, chromatiques, résistantes aux lavages, la transpiration, le sébum et à la lumière et de plus durables sans altération desdites fibres. De plus les colorations obtenues à partir du procédé donnent des couleurs

homogènes de la racine à la pointe d'une fibre (faible sélectivité de coloration).

*i) dérivé d'orthodiphénol :*

Un mode particulier de l'invention concerne des dérivés d'orthodiphénols ou mélanges de composés comprenant au moins un cycle aromatique, de préférence un cycle benzénique, comportant au moins deux groupes hydroxy (OH) portés par deux atomes de carbone adjacents du cycle aromatique. Particulièrement le ou les dérivés d'orthodiphénols selon l'invention ne sont pas des dérivés autoxydables à motif indole. Plus particulièrement ils sont différents du 5,6-dihydroxyindole.

Le cycle aromatique peut être plus particulièrement un cycle aryle condensé ou hétéroaromatique condensé i.e. contenant éventuellement un ou plusieurs hétéroatomes, tel que le benzène, le naphtalène, le tétrahydronaphtalène, l'indane, l'indène, l'anthracène, le phénanthrène, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine, ledit cycle aromatique comportant au moins deux groupes hydroxy portés par deux atomes de carbone adjacents du cycle aromatique. Préférentiellement le cycle aromatique des dérivés orthodiphénols selon l'invention est un cycle benzénique.

Par cycle condensé on entend qu'au moins deux cycles saturés ou insaturés, hétérocycliques ou non, présentent une liaison commune i.e. qu'au moins un cycle soit accolé à un autre cycle.

Les orthodiphénols selon l'invention peuvent être salifiés ou non. Ils peuvent également se trouver sous forme d'aglygone (sans sucre liés) ou sous forme de composés glycosylés.

Plus particulièrement le dérivé d'orthodiphénol i) représente un composé de formule (I), ou l'un de ces oligomères, sous forme salifiée ou non :

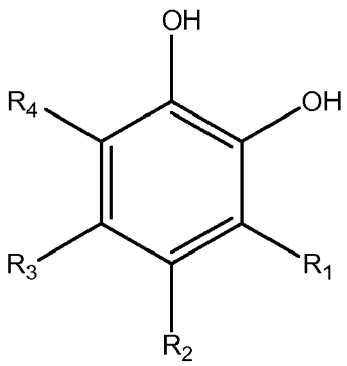

(I)

formule (I) dans laquelle les substituants :

- $R_1$ à $R_4$, identiques ou différents, représentent :
  - un atome d'hydrogène,
  - un atome halogène,
  - un radical hydroxy,
  - un radical carboxyle,
  - un radical carboxylate d'alkyle ou alcoxycarbonyle,
  - un radical amino éventuellement substitué,
  - un radical alkyle linéaire ou ramifié éventuellement substitué,
  - un radical alcényle linéaire ou ramifié éventuellement substitué,
  - un radical cycloalkyle éventuellement substitué,
  - un radical alcoxy,
  - un radical alcoxyalkyle,
  - un radical alcoxyaryle, le groupe aryle pouvant être éventuellement substitué,
  - un radical aryle,
  - un radical aryle substitué,
  - un radical hétérocyclique, saturé ou non, porteur ou non d'une charge cationique ou anionique, éventuellement substitué et/ou éventuellement condensé avec un cycle aromatique de préférence benzénique, ledit cycle

aromatique étant éventuellement substitué particulièrement par un ou plusieurs groupements hydroxy ou glycosyloxy,

- un radical contenant un ou plusieurs atomes de silicium,

• où deux des substituants portés par deux atome de carbone adjacents $R_1$ - $R_2$, $R_2$- $R_3$ ou $R_3$ - $R_4$ forment conjointement avec les atomes de carbone les portant un cycle saturé ou insaturé, aromatique ou non, contenant éventuellement un ou plusieurs hétéroatomes et éventuellement condensé avec un ou plusieurs cycles saturés ou insaturés contenant éventuellement un ou plusieurs hétéroatomes. Particulièrement $R_1$ à $R_4$ forment conjointement de un à quatre cycles.

Un mode de réalisation particulier de l'invention concerne des dérivés d'orthodiphénols de formule (I) dont deux substituants adjacents $R_1$- $R_2$, $R_2$- $R_3$ ou $R_3$- $R_4$ ne peuvent former avec les atomes de carbone qui les portent un radical pyrrolyle. Plus particulièrement $R_2$ et $R_3$ ne peuvent former un radical pyrrolyle condensé au cycle benzénique portant les deux hydroxy.

Les cycles saturés ou insaturés, éventuellement condensés, peuvent être aussi éventuellement substitués.

Les radicaux alkyles sont des radicaux hydrocarbonés, saturés, linéaires ou ramifiés, généralement en $C_1$-$C_{20}$, particulièrement en $C_1$-$C_{10}$, de préférence les radicaux alkyles en $C_1$-$C_6$, tels que méthyle, éthyle, propyle, butyle, pentyle et hexyle.

Les radicaux alcényles sont des radicaux hydrocarbonés, linéaires ou ramifiés, insaturés en $C_2$-$C_{20}$ ; de préférence comprenant au moins une double liaison tels que éthylène, propylène, butylène, pentylène, méthyl-2-propylène et décylène.

Les radicaux aryles sont des radicaux carbonés mono ou polycycliques, condensés ou non, comprenant préférentiellement de 6 à 30 atomes de carbone et dont au moins un cycle est aromatique ; préférentiellement choisis parmi le radical aryle est un phényle, biphényle, naphtyle, indényle, anthracényle, et tétrahydronaphtyle.

Les radicaux alcoxy sont des radicaux alkyle-oxy avec alkyle tel que défini précédemment, de préférence en $C_1$-$C_{10}$, tels que méthoxy, éthoxy, propoxy et butoxy.

Les radicaux alcoxy alkyles sont de préférence les radicaux alcoxy ($C_1$-$C_{20}$) alkyle ($C_1$-$C_{20}$), tels que méthoxyméthyle, éthoxyméthyle, méthoxyéthyle, éthoxyéthyle, etc.

Les radicaux cycloalkyles sont en général les radicaux cycloalkyles en $C_4$-$C_8$, de préférence les radicaux cyclopentyle et cyclohexyle. Les radicaux cycloalkyles peuvent être des radicaux cycloalkyles substitués, en particulier par des groupes alkyles, alcoxy, acide carboxylique, hydroxy, amine et cétone.

Les radicaux alkyles ou alcényles lorsqu'ils sont éventuellement substitués, peuvent être substitués par au moins un substituant porté par au moins un atome de carbone, choisi parmi :

- un atome d'halogène;
- un groupement hydroxy ;
- un radical alkoxy en $C_1$-$C_2$ ;
- un radical alcoxycarbonyle en $C_1$-$C_{10}$ ;
- un radical (poly)-hydroxyalkoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  * un groupement hydroxy,
  * un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
  * un groupement ammonium quaternaire $-N^+R'R''R'''$, M- pour lequel R', R'', R''', identiques ou différents représentent un atome d'hydrogène, ou un groupement alkyle en $C_1$-$C_4$; et M- représente le contre-ion de l'acide organique, minéral ou de l'halogénure correspondant,
  * ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$

éventuellement porteur d'au moins un groupement hydroxy et le radical R' est un radical alkyle en $C_1$-$C_2$ ; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino (R'$SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,

- un radical carboxylique sous forme acide ou salifiée (de préférence avec un métal alcalin ou un ammonium, substitué ou non) ;
- un groupement cyano ;
- un groupement nitro ;
- un groupement carboxy ou glycosylcarbonyle ;
- un groupement phénylcarbonyloxy éventuellement substitué par un ou plusieurs groupements hydroxy ;
- un groupement glycosyloxy ; et
- un groupement phényle éventuellement substitué par un ou plusieurs groupements hydroxy.

Les radicaux aryles ou hétérocycliques ou la partie aryle ou hétérocycliques des radicaux lorsqu'ils sont éventuellement substitués peuvent être substitués par au moins un substituant porté par au moins un atome de carbone, choisi parmi :

- un radical alkyle en $C_1$-$C_{10}$, de préférence en $C_1$-$C_8$, éventuellement substitué par un ou plusieurs radicaux choisis parmi les radicaux hydroxyle, alkoxy en $C_1$-$C_2$, (poly)-hydroxyalkoxy en $C_2$-$C_4$, acylamino, amino substitué par deux radicaux alkyle, identiques ou différents, en $C_1$-$C_4$, éventuellement porteurs d'au moins un groupement hydroxy ou, les deux radicaux pouvant former avec l'atome d'azote auquel ils sont rattachés, un hétérocycle comprenant de 5 à 7 chaînons, de préférence de 5 ou 6 chaînons, saturé ou insaturé éventuellement substitué comprenant éventuellement un autre hétéroatome identique ou différent de l'azote ;
- un atome d'halogène;
- un groupement hydroxy ;
- un radical alkoxy en $C_1$-$C_2$ ;
- un radical alcoxycarbonyle en $C_1$-$C_{10}$ ;
- un radical (poly)-hydroxyalkoxy en $C_2$-$C_4$ ;
- un radical amino ;
- un radical héterocycloalkyle à 5 ou 6 chaînons ;
- un radical hétéroaryle à 5 ou 6 chaînons éventuellement cationique, préférentiellement imidazolium, et éventuellement substitué par un radical ($C_1$-$C_4$) alkyle, préférentiellement méthyle ;
- un radical amino substitué par un ou deux radicaux alkyle, identiques ou différents, en $C_1$-$C_6$ éventuellement porteurs d'au moins :

  * un groupement hydroxy,
  * un groupement amino éventuellement substitué par un ou deux radicaux alkyle en $C_1$-$C_3$ éventuellement substitués, lesdits radicaux alkyle pouvant former avec l'atome d'azote auquel ils sont rattachés, un hétérocycle comprenant de 5 à 7 chaînons, saturé ou insaturé éventuellement substitué comprenant éventuellement au moins un autre hétéroatome différent ou non de l'azote,
  * un groupement ammonium quaternaire $-N^+R'R''R'''$, M- pour lequel R', R", R"', identiques ou différents représentent un atome d'hydrogène, ou un groupement alkyle en $C_1$-$C_4$; et M- représente le contre-ion de l'acide organique, minéral ou de l'halogénure correspondant,
  * ou un radical hétéroaryle à 5 ou 6 chaînons éventuellement cationique, préférentiellement imidazolium, et éventuellement substitué par un radical ($C_1$-$C_4$) alkyle, préférentiellement méthyle ;

- un radical acylamino (-NR-COR') dans lequel le radical R est un atome d'hydrogène, un radical alkyle en $C_1$-$C_4$ éventuellement porteur d'au moins un groupement hydroxy et le radical R' est un radical alkyle en $C_1$-$C_2$ ; un radical carbamoyle ($(R)_2N$-CO-) dans lequel les radicaux R, identiques ou non, représentent un atome d'hydrogène, un radical alkyle en $C_1$-$C_4$ éventuellement porteur d'au moins un groupement hydroxy ; un radical alkylsulfonylamino (R'$SO_2$-NR-) dans lequel le radical R représente un atome d'hydrogène, un radical alkyle en $C_1$-$C_4$ éventuellement porteur d'au moins un groupement hydroxy et le radical R' représente un radical alkyle en $C_1$-$C_4$, un radical phényle ; un radical aminosulfonyle ($(R)_2N$-$SO_2$-) dans lequel les radicaux R, identiques ou non, représentent un atome d'hydrogène, un radical alkyle en $C_1$-$C_4$ éventuellement porteur d'au moins un groupement hydroxy,
- un radical carboxylique sous forme acide ou salifiée (de préférence avec un métal alcalin ou un ammonium, substitué

ou non) ;

- un groupement cyano ;
- un groupement nitro ;
- un groupement polyhalogénoalkyle, préférentiellement le trifluorométhyle ;
- un groupement carboxy ou glycosylcarbonyle ;
- un groupement phénylcarbonyloxy éventuellement substitué par un ou plusieurs groupements hydroxy ;
- un groupement glycosyloxy ; et
- un groupement phényle éventuellement substitué par un ou plusieurs groupements hydroxy.

Par radical glycosyle on entend un radical issu d'un mono ou polysacharride.

Les radicaux contenant un ou plusieurs atomes de silicium sont de préférence des radicaux poly-diméthylsiloxane, poly-diphénylsiloxane, poly-diméthylphénylsiloxane, stéaroxy-diméthicone.

Les radicaux hétérocycliques sont en général des radicaux comprenant dans au moins un cycle un ou plusieurs hétéroatomes choisis parmi O, N et S, de préférence O ou N, éventuellement substitués par notamment un ou plusieurs groupes alkyles, alcoxy, acide carboxylique, hydroxy, amine ou cétone. Ces cycles peuvent contenir un ou plusieurs groupements oxo sur les atomes de carbone de l'hétérocycle.

Parmi les radicaux hétérocycliques utilisables, on peut citer les groupes furyle, pyrannyle, pyrrolyle, imidazolyle, pyrazolyle, pyridyle, thiényle.

De préférence encore, les groupes hétérocycliques sont des groupes condensés tels que des groupes benzofurannyle, chromènyle, xanthényle, indolyle, isoindolyle, quinolyle, isoquinolyle, chromannyle, isochromannyle, indolinyle, isoindolinyle, coumarinyle, isocoumarinyle, ces groupes pouvant être substitués, en particulier par un ou plusieurs groupes OH.

Les orthodiphénols utiles dans le procédé de l'invention peuvent être naturels ou synthétiques. Parmi les orthodiphénols naturels on inclut les composés qui peuvent être présents dans la nature et qui sont reproduits par (hémi) synthèse chimique.

Les sels des orthodiphénols de l'invention peuvent être des sels d'acides ou de bases. Les acides peuvent être minéraux ou organiques. De préférence l'acide est l'acide chlorhydrique qui conduit aux chlorures.

Les bases peuvent être minérales ou organiques. Particulièrement les bases sont des hydroxydes alcalins tels que la soude qui conduit à des sels de sodium.

Selon un mode de réalisation particulier de l'invention, la composition comprend comme ingrédient i) un ou plusieurs dérivé(s) d'orthodiphénol(s) synthétique(s) qui n'existe pas dans la nature.

Selon un autre mode de réalisation préféré de l'invention, le procédé de coloration des fibres kératiniques met en oeuvre comme ingrédient i) un ou plusieurs dérivé(s) d'orthodiphénol(s) naturel(s).

Plus particulièrement, les orthodiphénols utilisables dans le procédé de l'invention selon i) sont en particulier :

- les flavanols comme la catéchine et le gallate d'épichatéchine,
- les flavonols comme la quercétine,
- les anthocyanidines comme la cyanidine, la delphinidine, la pétunidine,
- les anthocyanines ou les anthocyanes comme la myrtilline ,
- les orthohydroxybenzoates, par exemple les sels d'acide gallique,
- les flavones comme la lutéoline,
- les hydroxystilbènes, par exemple le tétrahydroxy-3,3',4,5'-stilbène, éventuellement oxylés (par exemple glucosylés),
- la 3,4-dihydroxyphénylalanine et ses dérivés,
- la 2,3-dihydroxyphénylalanine et ses dérivés,
- la 4,5-dihydroxyphénylalanine et ses dérivés,
- les dihydroxycinnamates tels que l'acide caféique et l'acide chlorogénique,
- les orthopolyhydroxycoumarines,
- les orthopolyhydroxyisocoumarines,
- les orthopolyhydroxycoumarones,
- les orthopolyhydroxyisocoumarones,
- les orthopolyhydroxychalcones,
- les orthopolyhydroxychromones,
- les orthopolyhydroxyquinones,
- les orthopolyhydroxyxanthones,
- le 1,2 dihydroxybenzène et ses dérivés,
- le 1,2,4 trihydroxybenzène et ses dérivés,
- le 1,2,3 trihydroxybenzène et ses dérivés,

- le 2,4,5 trihydroxytoluène et ses dérivés,
- les proanthocyanidines et notamment les proanthocyanidines A1, A2, B1, B2, B3 et C1,
- les proanthocyanines,
- l'acide tannique,
- l'acide ellagique,
- et les mélanges des composés précédents.

Lorsque les précurseurs de coloration présentent des formes D et L, les deux formes peuvent être utilisées dans les compositions selon l'invention, ainsi que les racémiques.
Selon un mode de réalisation les orthodiphénols naturels sont issus d'extraits d'animaux, de bactéries, de champignons, d'algues, de plantes utilisés dans leur totalité ou partiellement. En particulier concernant les plantes les extraits sont issus de plante ou de partie de plantes telles que les fruits dont les agrumes, les légumes, les arbres, les arbustes. On peut aussi utiliser des mélanges de ces extraits, riches en othodiphénols tels que définis précédemment.
De préférence le ou les orthodiphénols naturels de l'invention sont issus d'extraits de plantes ou de parties de plantes
Au sens de l'invention on assimilera cesdits extraits dans leur ensemble en tant que composé i).
Les extraits sont obtenus par extraction de diverses parties de plantes telles que par exemple la racine, le bois, l'écorce, la feuille, la fleur, le fruit, le pépin, la gousse, la pelure.
Parmi les extraits de plantes, on peut citer les extraits de feuilles de thé, de rose..
Parmi les extraits de fruits, on peut citer les extraits de pomme, de raisin (en particulier de pépins de raisin), ou les extraits de fèves et/ou cabosses de cacaoyer.
Parmi les extraits de légumes, on peut citer les extraits de pomme de terre, de pelures d'oignon.
Parmi les extraits de bois d'arbres, on peut citer les extraits d'écorce de pin, les extraits de bois de campêche.
On peut également utiliser des mélanges d'extraits végétaux.
Selon un mode de réalisation particulier de l'invention le ou les dérivés d'orthodiphénols sont des extraits naturels, riches en orthodiphénols. Selon un mode préféré, le ou les dérivés d'orthodiphénols sont uniquement des extraits naturels.
Les extraits naturels selon l'invention peuvent se présenter sous forme de poudres ou de liquides. De préférence les extraits de l'invention se présentent sous forme des poudres.
Selon l'invention, le ou les dérivé(s) d'orthodiphénol(s) synthétique(s), naturel(s), et/ou le ou les extrait(s) naturel(s) utilisé(s) comme ingrédient i) dans une ou plusieurs composition(s) utile(s) dans le procédé selon l'invention représente(nt) de préférence, de 0.001% à 20% en poids du poids total de la ou des compositions contenant le ou les orthodiphénols ou le ou les extraits.
En ce qui concerne les orthodiphénols purs, la teneur dans la ou les compositions les contenant est comprise de préférence entre 0,001 et 5 % en poids de chacune de ces compositions.
En ce qui concerne les extraits, la teneur dans la ou les compositions contenant les extraits tels quels est comprise de préférence entre 0,5 et 20 % en poids de chacune de ces compositions.

*ii) sel métallique.*

Le procédé de l'invention utilise un ou plusieurs ingrédient(s) ii) qui sont des sel(s) métallique(s).
Particulièrement les sels métalliques sont choisis parmi les sels de Manganèse (Mn) et de Zinc (Zn).
Au sens de la présente invention on entend par sels, les oxydes de ces métaux et les sels proprement dits issus notamment de l'action d'un acide sur un métal. De préférence les sels ne sont pas des oxydes. Parmi les sels on peut citer les halogénures tels que les chlorures, fluorures et iodures ; les sulfates, les phosphates ; les nitrates ; les perchlorates et les sels d'acides carboxyliques et les complexes polymériques pouvant supporter lesdits sels ainsi que leurs mélanges.
Plus particulièrement le sel de manganèse est différent du carbonate de manganèse, de l'hydrogénocarbonate de manganèse ou du dihydrogénocarbonate de manganèse.
A titre d'exemple de complexes polymériques pouvant supporter lesdits sels polymériques on peut citer la pyrrolidone carboxylate de manganèse.
Les sels d'acides carboxyliques utilisables dans l'invention incluent également des sels d'acides carboxyliques hydroxylés tels que le gluconate.
A titre d'exemple, on peut citer le chlorure de manganèse, le fluorure de Mn, l'acétate de Mn tétrahydraté, le lactate de Mn trihydraté, le phosphate de Mn, l'iodure de Mn, le nitrate de Mn trihydraté, le bromure de Mn, le perchlorate de Mn tétrahydraté, le sulfate de Mn monohydraté et le gluconate de manganèse. Les sels utilisés avantageusement sont le gluconate de manganèse et le chlorure de manganèse.
Parmi les sels de zinc on peut citer le sulfate de zinc, le gluconate de zinc, le chlorure de zinc, le lactate de

zinc, l'acétate de zinc, le glycinate de zinc et l'aspartate de zinc.

Les sels de manganèse et de zinc peuvent être introduits sous forme solide dans les compositions ou bien provenir d'une eau naturelle, minérale ou thermale, riche en ces ions ou encore d'eau de mer (mer morte notamment). Ils peuvent aussi provenir de composés minéraux comme les terres, les ocres comme les argiles (argile verte par exemple) ou même d'extrait végétal les contenant (cf. par exemple brevet le document FR 2 814 943).

Particulièrement les sels métalliques de l'invention sont de degrés d'oxydation 2 tels que le Mn(II) et le Zn(II).

Selon un mode de réalisation préféré de l'invention, le ou les sels métalliques utilisés représentent de 0.001% à 10% en poids environ du poids total de la ou des composition(s) contenant ce ou ces sels métalliques et encore plus préférentiellement de 0,05% à 0,1% en poids environ.

*iii) peroxyde d'hydrogène ou système(s) générateur(s) de peroxyde d'hydrogène*

Dans le cadre de la présente invention, le troisième constituant est du peroxyde d'hydrogène ou un ou plusieurs système(s) générateur(s) de peroxyde d'hydrogène tel(s) que :

a) le peroxyde d'urée ;
b) les complexes polymériques pouvant libérer du peroxyde d'hydrogène tels que le polyvinylpyrrolidone/$H_2O_2$ en particulier se présentant sous forme de poudres et les autres complexes polymériques décrits dans US 5,008,093 ; US 3,376, 110 ; US 5,183,901 ;
c) les oxydases produisant du peroxyde d'hydrogène en présence d'un substrat adéquat (par exemple le glucose dans le cas de glucose oxydase ou l'acide urique avec l'uricase) ;
d) les peroxydes de métaux générant dans l'eau du peroxyde d'hydrogène comme le peroxyde de calcium, peroxyde de magnésium;
e) les perborates ; ou
f) les percarborates.

Selon un mode de réalisation préféré de l'invention la composition contient un ou plusieurs système(s) générateur(s) de peroxyde d'hydrogène choisi(s) parmi a) le peroxyde d'urée, b) les complexes polymériques pouvant libérer du peroxyde d'hydrogène choisis parmi le polyvinylpyrrolidone/$H_2O_2$ ; c) les oxydases ; e) les pérborates et f) le percarborates.

Particulièrement le troisième constituant est du peroxyde d'hydrogène.

Par ailleurs la ou les compositions comprenant le peroxyde d'hydrogène ou le ou les générateur(s) de peroxyde d'hydrogène peut ou peuvent également renfermer divers adjuvants utilisés classiquement dans les compositions pour la teinture des cheveux et tels que définis ci-après au point vi).

Selon un mode particulier de l'invention, le peroxyde d'hydrogène ou le ou les système(s) générateur(s) de peroxyde d'hydrogène utilisé(s) représente(nt) de préférence, de 0.001 % à 12% en poids exprimé en peroxyde d'hydrogène par rapport au poids total de la composition ou des compositions le ou les contenant et encore plus préférentiellement de 0,2% à 2,7% en poids.

*iv) (bi)carbonate(s)*

Dans le cadre de la présente invention, le quatrième ingrédient est choisi parmi les (bi)carbonates.

Par (bi)carbonates est sous entendu :

a) les carbonates de métal alcalins ($Mét_2^+$, $CO_3^{2-}$), de métal alcalino-terreux ($Mét'^{2+}$, $CO_3^{2-}$) d'ammonium ($(R''_4N+)_2$, $CO_3^{2-}$) ou de phosphonium ($(R''_4P^+)_2$,$CO_3^{2-}$ avec Mét' représentant un métal alcalino-terreux et Mét représentant un métal alcalin, et R", identiques ou différents, représentent un atome d'hydrogène, un groupement ($C_1$-$C_6$)alkyle éventuellement substitué tel que hydroxyéthyle),
et
b) les bicarbonates, également appelés hydrogénocarbonates, de formules suivantes :

➢ $R'^+$, $HCO_3$ avec R' représentant un atome d'hydrogène, un métal alcalin, un groupement ammonium $R''_4N^+$- ou phosphonium $R''_4P^+$- où R", identiques ou différents, représentent un atome d'hydrogène, un groupement ($C_1$-$C_6$)alkyle éventuellement substitué tel qu'hydroxyéthyle et lorsque R' représente un atome d'hydrogène l'hydrogénocarbonate est alors appelé dihydrogénocarbonate ($CO_2$, $H_2O$) ; et
➢ $Mét'^{2+}$ $(HC0_3^-)_2$ avec Mét' représentant un métal alcalino-terreux.

Plus particulièrement, le quatrième ingrédient est choisi parmi les (bi)carbonates de métal alcalin ou alcalino-terreux ; préférentiellement les (bi)carbonates de métal alcalin.

On peut citer les carbonates ou hydrogénocarbonates de Na, K, Mg, Ca et leurs mélanges, et en particulier l'hydrogénocarbonate de Na. Ces hydrogénocarbonates peuvent provenir d'une eau naturelle, par exemple eau de source du bassin de Vichy, de La Roche Posay, eau de Badoit (cf. brevet par exemple le document FR 2 814 943). Particulièrement on peut citer le carbonate de sodium [497-19-8] = $Na_2CO_3$, l'hydrogénocarbonate de sodium ou bicarbonate de soude [144-55-8] = $NaHCO_3$, et le dihydrogénocarbonate de sodium = $Na(HCO_3)_2$.

Selon l'invention, le ou les agents (bi)carbonates utilisés représentent de préférence, de 0.001% à 10% en poids du poids total de la ou des compositions contenant le ou les agents (bi)carbonates et encore plus préférentiellement de 0,005% à 5% en poids.

*v) l'eau:*

Selon un mode réalisation de l'invention, de l'eau est de préférence incluse dans le procédé de l'invention. Elle peut provenir de l'humidification des fibres kératiniques et/ou de la ou des compositions comprenant les composés i) à iv) tels que définis précédemment ou d'une ou plusieurs autres compositions. De préférence l'eau provient au moins d'une composition comprenant au moins un composé choisi parmi i) à iv) tels que définis précédemment.

*vi) compositions cosmétiques:*

Les compositions cosmétiques selon l'invention sont cosmétiquement acceptable i.e. elles comprennent un support de coloration qui contient généralement de l'eau ou un mélange d'eau et d'un ou plusieurs solvants organiques ou un mélange de solvants organiques.

Par solvant organique, on entend une substance organique capable de dissoudre ou disperser une autre substance sans la modifier chimiquement.

*Les Solvants organiques:*

A titre de solvant organique, on peut par exemple citer les alcanols inférieurs en $C_1$-$C_4$, tels que l'éthanol et l'isopropanol ; les polyols et éthers de polyols comme le 2-butoxyéthanol, le propylèneglycol, le monométhyléther de propylèneglycol, le monoéthyléther et le monométhyléther du diéthylèneglycol, l'hexylène glycol, ainsi que les alcools aromatiques comme l'alcool benzylique ou le phénoxyéthanol.

Les solvants organiques sont présents dans des proportions de préférence comprises entre 1 et 40 % en poids environ par rapport au poids total de la composition tinctoriale, et encore plus préférentiellement entre 5 et 30 % en poids environ.

*Les Adjuvants:*

La ou les compositions du procédé de coloration conforme à l'invention peut également renfermer divers adjuvants utilisés classiquement dans les compositions pour la teinture des cheveux, tels que des agents tensio-actifs anioniques, cationiques, non-ioniques, amphotères, zwitterioniques ou leurs mélanges, des polymères anioniques, cationiques, non-ioniques, amphotères, zwitterioniques ou leurs mélanges, des agents épaississants minéraux ou organiques, et en particulier les épaississants associatifs polymères anioniques, cationiques, non ioniques et amphotères, des agents antioxydants, des agents de pénétration, des agents séquestrants, des parfums, des tampons, des agents dispersants, des agents de conditionnement tels que par exemple des silicones volatiles ou non volatiles, modifiées ou non modifiées, des agents filmogènes, des céramides, des agents conservateurs, des agents opacifiant.

Lesdits adjuvants sont choisis de préférence parmi des agents tensioactifs tels que des tensioactifs anioniques, non ioniques ou leurs mélanges et des agents épaississants minéraux ou organiques.

Les adjuvants ci-dessus sont en général présents en quantité comprise pour chacun d'eux entre 0,01 et 40 % en poids par rapport au poids de la composition, de préférence entre 0,1 et 20 % en poids par rapport au poids de la composition.

Bien entendu, l'homme de l'art veillera à choisir ce ou ces éventuels composés complémentaires de manière telle que les propriétés avantageuses attachées intrinsèquement à la composition ou aux composition(s) utiles dans le procédé de coloration conforme à l'invention ne soient pas, ou substantiellement pas, altérées par la ou les adjonctions envisagées.

*Les Colorants additionnels:*

Le procédé mettant en oeuvre les ingrédients i) à v) tels que définis précédemment ou la composition cosmétique

selon l'invention comprenant les ingrédients i) à v) tels que définis précédemment peut en outre mettre en oeuvre ou comprendre un ou plusieurs colorants directs additionnels. Ces colorants directs sont par exemple choisis parmi ceux classiquement utilisés en coloration directe, et parmi lesquels on peut citer tous les colorants aromatiques et/ou non aromatiques d'utilisation courante tels que les colorants directs nitrés benzéniques neutres, acides ou cationiques, les colorants directs azoïques neutres, acides ou cationiques, les colorants directs naturels autres que les orthodiphénols, les colorants directs quinoniques et en particulier anthraquinoniques neutres, acides ou cationiques, les colorants directs aziniques, triarylméthaniques, indoaminiques, les méthines, les styriles, les porphyrines, métalloporphyrines, les phtalocyanines, les cyanines méthiniques, et les colorants fluorescents. Tous ces colorants additionnels sont différents des dérivés d'orthodiphénols selon l'invention.

Parmi les colorants directs naturels, on peut citer la lawsone, la juglone, l'indigo, l'isatine, la curcumine, la spinulosine, l'apigénidine, les orcéines. On peut également utiliser les extraits ou décoctions contenant ces colorants naturels et notamment les cataplasmes ou extraits à base de henné.

Le ou les colorants directs additionnels utilisés dans la ou les compositions représentent de préférence, de 0.001% à 10% en poids environ du poids total de la ou des compositions les contenant et encore plus préférentiellement de 0.05% à 5% en poids environ.

Les compositions du procédé mettant en oeuvre les ingrédients i) à v) tels que définis précédemment ou la composition cosmétique selon l'invention comprenant les ingrédients i) à v) tels que définis précédemment peut également mettre en oeuvre ou comprendre une ou plusieurs bases d'oxydation et/ou un ou plusieurs coupleurs conventionnellement utilisés pour la teinture de fibres kératiniques.

Parmi les bases d'oxydation, on peut citer les para-phénylènediamines, les bis-phénylalkylènediamines, les para-aminophénols, les bis-para-aminophénols, les ortho-aminophénols, les bases hétérocycliques et leurs sels d'addition

Parmi ces coupleurs, on peut notamment citer les méta-phénylènediamines, les méta-aminophénols, les méta-diphénols, les coupleurs naphtaléniques, les coupleurs hétérocycliques et leur sels d'addition.

La ou les bases d'oxydation présentes dans la ou les compositions sont en général présentes chacune en quantité comprise entre 0,001 à 10 % en poids du poids total de la ou des compositions correspondantes.

La ou les composition(s) cosmétique(s) de l'invention peuvent se présenter sous des formes galéniques diverses, telles qu'une poudre, une lotion, une mousse, une crème, un gel ou sous tout autre forme appropriée pour réaliser une teinture des fibres kératiniques. Elles peuvent également être conditionnées en flacon pompe sans propulseur ou sous pression en flacon aérosol en présence d'un agent propulseur et former une mousse.

*pH de la ou des composition(s)*

Selon un mode particulier de l'invention le pH de la ou des compositions contenant le ou les (bi)carbonates est supérieur à 7 et de préférence compris entre 8 et 12. Particulièrement compris entre 8 et 10.

Si la ou les compositions, ne contiennent pas de (bi)carbonates, le pH de la ou des compositions contenant le peroxyde d'hydrogène ou un système générateur de peroxyde d'hydrogène, est de préférence inférieur à 7, plus particulièrement compris entre 1 et 5.

De préférence la ou les compositions contenant le ou les orthodiphénols de l'invention et ne contenant pas de (bi)carbonates sont à pH intérieur à 7 et de préférence comprise entre 3 et 6,5.

Selon un mode particulier de l'invention les compositions contenant le ou les sels métalliques et ne contenant de (bi)carbonates sont à pH inférieur à 7 et de préférence comprise entre 3 et 6,5.

Le pH de ces compositions peut être ajustés à la valeur désirée au moyen d'agents acidifiants ou alcalinisants habituellement utilisés en teinture des fibres kératiniques ou bien encore à l'aide de systèmes tampons classiques.

Parmi les agents acidifiants des compositions utilisées dans l'invention, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Une variante avantageuse est d'ajouter un agent alcalinisant à la ou les compositions du procédé de coloration contenant le ou les (bi)carbonates. Plus particulièrement, cet agent alcalin est choisi parmi l'ammoniaque, les carbonates alcalins, les alcanolamines telles que les mono-, di- et triéthanolamines ainsi que leurs dérivés, les hydroxydes de sodium ou de potassium et les composés de formule (II) suivante :

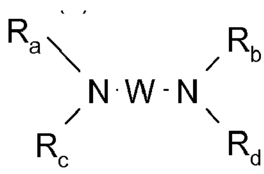

(II)

dans laquelle W est un reste propylène éventuellement substitué par un groupement hydroxy ou un radical alkyle en $C_1$-$C_4$ ; $R_a$, $R_b$, $R_c$ et $R_d$, identiques ou différents, représentent un atome d'hydrogène, un radical alkyle en $C_1$-$C_4$ ou hydroxyalkyle en $C_1$-$C_4$.

*vii) Procédé de coloration en une ou plusieurs étapes*

Selon un mode de réalisation particulier de l'invention le procédé de coloration est réalisé, en une ou plusieurs étapes, par application sur les fibres kératiniques d'une ou de plusieurs compositions cosmétiques contenant, pris ensemble ou séparément dans la ou les dites compositions, les ingrédients suivants:

i) un ou plusieurs dérivé(s) d'orthodiphénol(s),
ii) un ou plusieurs sel(s) métallique(s), de préférence choisis parmi les sels de Mn et de Zn,
iii) du peroxyde d'hydrogène ou un ou plusieurs système(s) générateur(s) de peroxyde d'hydrogène ; et
iv) un ou plusieurs (bi)carbonate(s) ou un ou plusieurs système(s) générateur(s) de (bi)carbonate(s),

étant entendu qu'au moins une desdites compositions est aqueuse.

Le temps de pause entre les étapes d'application des compositions comprenant le ou les ingrédients i), ii), iii) et/ou iv) est fixé entre 3 et 120 minutes, préférentiellement entre 10 et 60 minutes et plus particulièrement entre 15 et 45 minutes.

Les fibres kératiniques peuvent être ou non préalablement humidifiées.

Plus particulièrement, dans le procédé de l'invention le ou les composé(s) iv) se trouvent :

- soit en mélange avec les ingrédients i), ii) et iii)
- soit appliqué séparément après application d'une composition cosmétique comprenant les ingrédients i), ii) et iii) ; ou alors
- soit appliqué ensemble avec l'ingrédient iii) après application d'une composition cosmétique comprenant les ingrédients i) et ii).

Un mode de réalisation particulier de l'invention concerne les procédés de coloration en une ou deux étapes.

Selon un mode particulier de l'invention le procédé de coloration des fibres kératiniques est réalisé en une seule étape par l'application sur les fibres kératiniques d'une composition cosmétique colorante aqueuse comprenant i), ii), iii) et iv) tels que définis précédemment..

Le temps de pause après application est généralement fixé entre 3 et 120 minutes et préférentiellement entre 10 et 60 minutes et plus préférentiellement entre 15 et 45 minutes.

Selon un autre mode de réalisation particulier de l'invention, le procédé de coloration de fibres kératiniques se fait en deux étapes.

Dans une première variante de procédé en deux étapes la première étape consiste à appliquer sur les dites fibres une composition cosmétique comprenant les ingrédients i), ii), iii) tels que définis précédemment, puis dans une deuxième étape une composition cosmétique comprenant l'ingrédient iv) tel que défini précédemment est appliquée sur lesdites fibres, étant entendu qu'au moins une des deux compositions cosmétiques est aqueuse.

Dans une deuxième variante de procédé de coloration de fibres kératiniques en deux étapes, la première étape consiste à appliquer sur les dites fibres une composition comprenant les ingrédients i) et ii), tels que définis précédemment, puis dans une deuxième étape une seconde composition cosmétique comprenant les ingrédients iii) et iv) tels que définis précédemment est appliquée sur lesdites fibres, étant entendu que de préférence au moins une des deux compositions est aqueuse.

Selon un procédé de coloration particulier de l'invention, ledit procédé est réalisé en au moins deux étapes se terminant par le traitement des fibres kératiniques avec l'ingrédient iv) et peut être suivi d'étapes de post- traitement tel que d'un shampooinage à l'aide de shampoing classique, d'un rinçage par exemple à l'eau et/ou du séchage des fibres kératiniques par traitement thermique tel que défini ci après; étant entendu que ledit procédé ne fait pas intervenir de rinçage intermédiaire juste avant l'étape qui met en oeuvre l'ingrédient iv).

Préférentiellement, le procédé de coloration selon l'invention est réalisé en deux étapes dont la première étape est d'appliquer sur les fibres kératiniques les ingrédients i) et ii) ensemble puis dans une deuxième étape d'appliquer ensemble les ingrédients iii) et iv) ou dans une première étape d'appliquer ensemble les ingrédients i), ii) et iii) puis dans une deuxième étape d'appliquer iv). Ces procédés peuvent être suivis d'étapes de post-traitement tel que le rinçage par exemple à l'eau, le shampooinage avec un shampoing classique et/ou le séchage des fibres kératiniques. Préférentiellement, le procédé selon l'invention en au moins deux étapes ne fait pas intervenir de rinçage intermédiaire entre la première et la deuxième étape i.e. entre l'application du mélange des ingrédients i), ii), iii) et iv) ou entre l'application du mélange i), ii) et du mélange iii), iv).

Selon un procédé particulièrement avantageux les fibres kératiniques sont juste avant l'étape qui met en oeuvre l'ingrédient iv) :

a) soit essuyé mécaniquement tel que décrit ci après,
b) soit séchées par la chaleur avec un traitement thermique tel que décrit ci après,
c) soit non rincées c'est-à-dire que les étapes se font successivement. De façon préférée entre la première et la deuxième étape du procédé de coloration de l'invention les fibres sont :

  a) soit essuyé mécaniquement tel que décrit ci après,
  b) soit séchées par la chaleur avec un traitement thermique tel que décrit ci après,
  c) soit non rincées c'est-à-dire que les étapes 1 et 2 se font successivement.

Selon un procédé particulièrement préféré de l'invention juste avant l'étape qui met en oeuvre l'ingrédient iv) les fibres sont a) essuyées mécaniquement.

Plus préférentiellement entre la première et deuxième étape les fibres sont essuyées préférentiellement à l'aide d'une serviette ou d'un papier absorbant, ou sont séchées par la chaleur avec un traitement thermique à une température comprise particulièrement entre 60 et 220°C et de préférence entre 120 et 200°C.

Selon un autre mode particulier de l'invention entre l'application du mélange des ingrédients i), ii), iii) et iv) ou entre l'application du mélange i), ii) et du mélange iii), iv), les mèches sont rincées très rapidement, entre 1 seconde et 1 minute plus particulièrement entre 1 seconde et 30 seconde, et préférentiellement entre 2 et 5 secondes tel que 2 secondes, sous à l'eau d'un robinet ou douchette à jet intense. Cette étape de rinçage rapide est suivie d'un essuyage mécanique tel que décrit ci après.

Pour ces procédés le temps de pause après application de la composition cosmétique pour la première étape est généralement fixé entre 3 et 120 minutes et préférentiellement entre 10 et 60 minutes et plus préférentiellement entre 15 et 45 minutes. Le temps de pause après application de la deuxième composition cosmétique pour la deuxième étape est généralement fixé entre 3 et 120 minutes et préférentiellement entre 3 et 60 minutes et plus préférentiellement entre 5 et 30 minutes.

Quelque soit le mode d'application, la température d'application est généralement comprise entre la température ambiante (15 à 25°C) et 80°C et plus particulièrement entre 15 et 45°C. Ainsi, on peut, avantageusement, après application de la composition selon l'invention, soumettre la chevelure à un traitement thermique par chauffage à une température comprise entre 30 et 60°C. Dans la pratique, cette opération peut être conduite au moyen d'un casque de coiffure, d'un sèche-cheveux, d'un dispensateur de rayons infrarouges et d'autres appareils chauffants classiques.

On peut utiliser, à la fois comme moyen de chauffage et de lissage de la chevelure, un fer chauffant à une température comprise entre 60 et 220°C et de préférence entre 120 et 200°C.

Un mode particulier de l'invention concerne un procédé de coloration qui est réalisé à température ambiante (25°C).

Dans tous les modes particuliers et variantes des procédés précédemment décrits les compositions évoquées sont des compositions prêtes à l'emploi qui peuvent résulter du mélange extemporané de deux ou plusieurs compositions et notamment de compositions présentes dans des kits de teintures.

*viii) étape(s) d'essuyage mécanique et/ou séchage :*

Selon un mode particulier de l'invention, le procédé de coloration des fibres kératiniques comprend au moins une étape intermédiaire d'essuyage mécanique des fibres et/ou de séchage et/ou non rincées. Les étapes d'essuyage mécanique et de séchage intermédiaire sont également appelées « non rincé maitrisé » pour se différencier du « rinçage classique à grande eau » et du « non rincé ».

Par essuyage mécanique des fibres on entend le frottement d'un objet absorbant sur les fibres et le retrait physique par l'objet absorbant du surplus d'ingrédient(s) n'ayant pas pénétré dans les fibres. L'objet absorbant peut être une pièce de tissu comme une serviette particulièrement éponge, un torchon, du papier absorbant tel que de l'essuie-tout.

Selon un procédé particulièrement avantageux de l'invention, l'essuyage mécanique est effectué sans séchage total de la fibre, laissant la fibre humide.

Par séchage on entend l'action d'évaporer des solvants organiques et/ou de l'eau se trouvant dans une ou plusieurs compositions utilisées dans le procédé de l'invention, comprenant ou non un ou plusieurs ingrédients i à iv) tels que définis précédemment. Le séchage peut se faire par source thermique (convection, conduction ou rayonnement) en envoyant par exemple un courant gazeux chaud tel que l'air nécessaire à l'évaporation du ou des solvants. A titre de source thermique on peut citer un sèche-cheveux, de casques à cheveux, d'un fer à lisser les cheveux, d'un dispensateur de rayons infrarouges et d'autres appareils chauffants classiques.

*ix) Dispositif ou « kit » de teinture :*

Un autre objet de l'invention est un dispositif à plusieurs compartiments ou « kit » de teinture. De façon avantageuse, ce kit comporte de 2 à 5 compartiments contenant de 2 à 5 compositions dans lesquels sont répartis les ingrédients i) un ou plusieurs dérivé(s) d'orthodiphénol(s), ii) un ou plusieurs sel(s) métallique(s), iii) du peroxyde d'hydrogène ou un ou plusieurs système(s) générateur(s) de peroxyde d'hydrogène et iv) un ou plusieurs (bi)carbonate(s), lesdites compositions étant aqueuses ou pulvérulentes, avec particulièrement au moins une de ces compositions étant aqueuse.

Selon une première variante le kit comporte cinq compartiments, les quatre premiers compartiments comprenant respectivement les ingrédients en poudres i), ii), iii) et iv) tels que définis précédemment et le cinquième compartiment contenant une composition aqueuse tel que de l'eau. Dans ce cas le ou les composés iii) sont des précurseurs de peroxyde d'hydrogène.

Une autre variante concerne un kit à quatre compartiments dont l'un d'entre eux au moins contient une composition aqueuse, et comprenant un ingrédient i) à iv) tels que définis précédemment.

Dans une autre variante le dispositif à quatre compartiments : le premier compartiment comprenant une composition cosmétique contenant i) un ou plusieurs dérivé(s) d'orthodiphénol(s), le deuxième compartiment comprenant une composition contenant ii) un ou plusieurs sel(s) métallique(s), le troisième compartiment comprenant une composition contenant iii) du peroxyde d'hydrogène ou un ou plusieurs système(s) générateur(s) de peroxyde d'hydrogène et le quatrième compartiment contenant une composition contenant iv) un ou plusieurs (bi)carbonate(s) ; l'une au moins de ces compositions étant de préférence aqueuse.

Un autre mode de réalisation préféré concerne un dispositif comprenant trois compartiments :

(a) un premier compartiment contient une composition renfermant :

  i) un ou plusieurs ortho diphénols ; et

(b) un deuxième compartiment contient une composition renfermant :

  ii) un ou plusieurs sel(s) métallique(s),
  iii) du peroxyde d'hydrogène ou un ou plusieurs système(s) générateur(s) de peroxyde d'hydrogène ;

(c) un troisième compartiment contient iv) un ou plusieurs (bi)carbonate(s)

Dans cet autre mode de réalisation, au moins une des trois compositions est de préférence aqueuse et le ou les orthodiphénols peuvent être sous forme de poudre.

On peut aussi avoir un kit à trois compartiments, le premier a) contenant une composition comprenant i) un ou plusieurs orthodiphénols et ii) un ou plusieurs sels métalliques, le second b) contenant une composition comprenant iii) le peroxyde d'hydrogène ou un système générateur de peroxyde d'hydrogène et le troisième c) contenant une composition comprenant iv) l'agent (bi)carbonate. Dans cet autre kit au moins une des compositions est de préférence aqueuse. Cette composition contient de préférence du peroxyde d'hydrogène.

Selon un mode particulier de l'invention, le kit comporte deux compartiments : un premier compartiment comprenant une composition contenant i) un ou plusieurs dérivé(s) d'orthodiphénol(s), ii) un ou plusieurs sel(s) métallique (s), iii) du peroxyde d'hydrogène ou un ou plusieurs système(s) générateur(s) de peroxyde d'hydrogène et un deuxième compartiment contenant iv) un ou plusieurs (bi)carbonate(s).

Parmi les kits à deux compartiments sont également possibles les kits qui contiennent dans un premier compartiment une composition comprenant les composés i), ii) et iv) tels que définis précédemment et dans un deuxième compartiment une composition comprenant le composé iii) tel que défini précédemment.

Dans ces deux variantes de kit à deux compartiments la première composition contenue dans le premier compartiment comprenant soit i), ii) et iii) ou i), ii) et iv) est sous forme de poudre et de préférence la seconde composition

est aqueuse.

Selon une variante, le dispositif selon l'invention, comprend, en outre, une composition supplémentaire (c) comprenant un ou plusieurs agents traitants.

Les compositions du dispositif selon l'invention sont conditionnées dans des compartiments distincts, accompagnés, éventuellement, de moyens d'application appropriés, identiques ou différents, tels que les pinceaux, les brosses ou les éponges.

Le dispositif mentionné ci-dessus peut également être équipé d'un moyen permettant de délivrer sur les cheveux le mélange souhaité, par exemple tel que les dispositifs décrits dans le brevet FR 2 586 913.

## EXEMPLES DE COLORATION

### Exemple 1 :

Les compositions suivantes ont été préparées :

| **Composition** | (**a1**) |
|---|---|
| Epicatéchine | 10g |
| Ethanol | 29g |
| Propylène glycol | 29g |
| Chlorure de manganèse | 0,05g |
| Hydroxypropyl cellulose | 0,5g |
| Peroxyde d'hydrogène | 1,2g |
| Eau déminéralisée | Qsp 100g |
| | |
| **Composition** | (**b1**) |
| Bicarbonate de sodium | 2,6g |
| Carbomer | 1g |
| Monoéthanolamine | Qsp pH 9 |
| Eau déminéralisée | Qsp 100g |

La composition (**a1**) est appliquée sur des cheveux secs naturels à 90% de blancs avec un rapport de bain de 5 g de formule pour 1g de cheveux. On laisse ensuite poser pendant 30 minutes à l'aide d'un casque chauffant à 45°C.

La composition (**b1**) est ensuite appliquée sur des cheveux pour un temps de pose de 10 minutes à température ambiante. Au bout de quelques minutes une coloration rouge cuivré très intense apparaît.

Après avoir rincé, shampouiné et séché, les cheveux sont tous les deux teints dans une nuance de cuivré intense. La coloration est très tenace aux lavages et à la lumière.

### Exemple 2:

Les compositions suivantes ont été préparées :

| **Compositions** | **(a2)** |
|---|---|
| Extrait de feuille de thé vert | 5g |
| Lauryl ether sulfate de sodium à 70% dans l'eau | 10g |
| Héxylène glycol | 5g |
| Gluconate de manganèse | 0,05g |
| Peroxyde d'hydrogène | 1,2g |
| Eau déminéralisée | Qsp 100g |

(suite)

| Compositions | (a2) |
|---|---|
| | |
| **Composition** | (**b1**) |
| Bicarbonate de sodium | 2,6g |
| Carbomer | 1g |
| Monoéthanolamine | Qsp pH 9 |
| Eau déminéralisée | Qsp 100g |

La composition (**a2**) est appliquée sur des cheveux secs naturels à 90% de blancs avec un rapport de bain de 5 g de formule pour 1g de cheveux. On laisse ensuite poser pendant 45 minutes à température ambiante.

La composition (**b1**) est ensuite appliquée sur des cheveux pour un temps de pose de 10 minutes à température ambiante.

Après avoir rincé, shampouiné et séché, les cheveux sont respectivement teints dans une nuance de léger doré naturel. La coloration est très tenace aux lavages et à la lumière.

**1) EXEMPLES COMPARATIFS**

Les compositions suivantes ont été préparées :

| **Composition A** | **A1 comparatif** | *A2 comparatif* | **A3 Invention** | *A4 Invention* | *A5 Invention* |
|---|---|---|---|---|---|
| Catéchine | 5g | - | 5g | - | - |
| Extrait de fèves de cacao | - | - | - | 5g | - |
| Extrait d'écorce de pin | - | 5g | - | - | 5g |
| Héxylène glycol | 5g | 5g | 5g | 5g | 5g |
| Lauryl ether sulfate de sodium (70% en MA) | 3,75g | 3,75g | 3,75g | 3,75g | 3,75g |
| Chlorure de manganèse tétrahydraté (soit 0,01% en poids d'équivalent métal $Mn^{2+}$) | 0,036g | 0,036g | 0,036g | - | 0,036g |
| Pyrrolidone carboxylate de manganèse (soit 0,006% en poids d'équivalent métal $M_n^{2+}$) | - | - | - | 0,062g | - |
| Peroxyde d'hydrogène | - | - | 1,2g | 1,2g | - |
| Peroxyde d'urée 35% (soit 1,2% en poids d'équivalent de peroxyde d'hydrogène) | - | - | - | - | 3,4g |
| Acide citrique ou hydroxyde de sodium | qsp pH 5 | qsp pH 5 | qsp pH 5 | qsp pH 5 | qsp pH 5 |
| Eau déminéralisée | qsp 100g | qsp 100g | qsp 100g | qsp 100g | qsp 100g |

La composition **A** est appliquée sur des mèches de cheveux secs à 90% de blancs naturels et à 90% de blancs permanentés avec un rapport de bain de 5 g de formule pour 1g de cheveux. On laisse ensuite poser pendant 30 minutes à une température de 50°C.

A l'issu, les cheveux imprégnés de la première composition sont essuyés à l'aide d'une serviette de papier

absorbant pour enlever l'excédant de formule.

| **Composition B** | **B1** |
|---|---|
| Bicarbonate de sodium : $NaHCO_3$ | 2,6g |
| Carbomer | 1g |
| Monoéthanolamine | qsp pH 9 |
| Eau déminéralisée | qsp 100g |

La composition **B** est ensuite appliquée sur les cheveux avec un rapport de bain de 4 g pour 1g de mèche ; le temps de pose est de 10 minutes à température ambiante. Au bout de quelques minutes une coloration très intense apparaît. Les cheveux sont ensuite rincés à l'eau, lavés avec un shampooing classique et séchés au casque.

**Résultats colorimétriques :**

La coloration des cheveux est évaluée visuellement et lue au spectrocolorimètre Minolta (CM3600d, illuminant D65, angle 10°, valeurs SCI) pour les mesures colorimétriques L*, a*, b*.
Dans ce système L* a* b*, L* représente l'intensité de la couleur, a* indique l'axe de couleur vert/rouge et b* l'axe de couleur bleu/jaune. Plus la valeur de L est faible, plus la couleur est foncée ou très intense. Plus la valeur de a* est élevée plus la nuance est rouge et plus la valeur de b* est élevée plus la nuance est jaune.
La variation de coloration entre les mèches colorées de cheveux blancs naturels/permanentés non traités (témoin) et après traitement sont définis par (ΔE*) selon l'équation suivante :

$$\Delta E^* = \sqrt{(L^* - L_o^*)^2 + (a^* - a_o^*)^2 + (b^* - b_o^*)^2}$$

Dans cette équation, L*, a* et b* représentent les valeurs mesurées après coloration des cheveux naturels/permanentés à 90% de blancs et $L^*_0$, $a_0^*$ et $b_0^*$ représentent les valeurs mesurées des cheveux naturels/permanentés à 90% non traités.
Plus la valeur de ΔE est importante, plus la différence de couleur entre les mèches témoins et les mèches colorées est importante.
La coloration est très tenace aux lavages et à la lumière.

| **Exemples** (sur cheveux à 90% de **blancs naturels**) | **Témoin** (cheveux non traités) | **3** | **4** | **5** | **6** | **7** |
|---|---|---|---|---|---|---|
| Composition (Ai) Etape 1 | - | **A1** | *A2* | **A3** | A4 | *A5* |
| Composition (Bi) Etape 2 | - | B1 | B1 | B1 | B1 | B1 |
| Nuances sur cheveux | - | très léger doré | très léger beige | cuivré très intense | cuivré très intense | cuivré doré |
| Intensité (L*) | 47,08 | **51,08** | 46,28 | **41,32** | 45,22 | 47,93 |
| a* | 1,9 | 3,31 | 3,48 | 11,2 | 8,94 | 7,54 |
| b* | 11,74 | 20,31 | 13,66 | 25,77 | 22,71 | 18,42 |
| ΔE* | - | **9,56** | 2,62 | **17,79** | 13,17 | 8,79 |
| ΔL* | - | 4 | -0,8 | -5,76 | -1,85 | 0,86 |
| Δa* | - | 1,41 | 1,59 | 9,3 | 7,04 | 5,64 |
| Δb* | - | 8,57 | 1,93 | 14,03 | 10,98 | 6,68 |

(suite)

| **Exemples** (sur cheveux à 90% de **blancs naturels**) | **Témoin** (cheveux non traités) | **3** | **4** | **5** | **6** | **7** |
|---|---|---|---|---|---|---|
| | | | | | | |
| **Exemples** (sur cheveux à 90% de blancs naturels **permanentés)** | **Témoin** (cheveux, non traités) | **8** | **9** | **10** | **11** | **12** |
| Composition (Ai) Etape 1 | - | **A1** | *A2* | **A3** | A4 | *A5* |
| Composition (Bi) Etape 2 | - | B1 | B1 | B1 | B1 | B1 |
| Nuances sur cheveux | - | très léger vert | très léger beige | cuivré très intense | cuivré très intense | cuivré doré |
| Intensité (L*) | 47,22 | **45,48** | 47,63 | **41,58** | 38,64 | 47,3 |
| a* | 0,31 | -0,06 | 1,16 | 9 | 10,39 | 6,58 |
| b* | 10,45 | 15,54 | 12,6 | 26,42 | 25,08 | 17,39 |
| ΔE* | - | **5,4** | *2,35* | **19,04** | *19,73* | *9,36* |
| ΔL* | - | -1,74 | 0,41 | -5,63 | -8,58 | 0,09 |
| Δa* | - | -0,37 | 0,85 | 8,69 | 10,08 | 6,27 |
| Δb* | - | 5,09 | 2,15 | 15,97 | 14,64 | 6,95 |

Il apparaît des tableaux ci-dessus que les mèches de cheveux blancs naturels ou permanentés traités avec la composition selon l'invention permet de colorer de façon significativement plus chromatique que la composition selon le comparatif (voir A1+B1 vs. A3+B1 ; et A2+B1 vs. A4+B1 et A5+B1). Par ailleurs les compositions selon l'invention procurent de la couleur aux cheveux beaucoup plus intense que celles obtenues avec les comparatifs (L* plus faible avec les compositions selon l'invention).

**<u>II) AUTRES EXEMPLES COMPARATIFS</u>**

Les compositions suivantes ont été préparées :

| **Composition A'** | **A'1 Invention** | **A'2 Comparatif** | ***A'3 Invention*** | ***A'4 Comparatif*** |
|---|---|---|---|---|
| Catéchine | 4g | 4g | 4g | 4g |
| Héxylène glycol | 5g | 5g | 5g | 5g |
| Lauryl ether sulfate de sodium (70% en MA) | 3,75g | 3,75g | 3,75g | 3,75g |
| Chlorure de manganèse tétrahydraté (soit 0,01% en poids d'équivalent métal $M_n^{2+}$) | 0,036g | - | *0,036g* | - |
| Peroxyde d'hydrogène | 1,2g | 1,2g | - | - |
| Acide citrique ou hydroxyde de sodium | qsp pH 5 | qsp pH 5 | qsp pH 5 | *qsp pH 5* |
| Eau déminéralisée | qsp 100g | qsp 100g | qsp 100g | *qsp 100g* |

La composition **A'** est appliquée sur des mèches de cheveux secs à 90% de blancs naturels avec un rapport de bain de 5 g de formule pour 1 g de cheveux. On laisse ensuite poser pendant 30 minutes à une température de 50°C.
A l'issu du temps de pose, les cheveux imprégnés de la première composition sont soit essuyés à l'aide d'une serviette de papier absorbant pour enlever l'excédant de formule, soit rincés puis essuyés.

| **Composition B'** | **B'1** | **B'2** | **B'3** | **B'4** |
|---|---|---|---|---|
| Bicarbonate de sodium $NaHCO_3$ | 2,6g | 2,6 g | 2,6 g | 2,6 g |
| Chlorure de manganèse tétrahydraté (soit 0,01% en poids d'équivalent métal $Mn^{2+}$) | - | 0,036 g | - | 0,036 g |
| Peroxyde de sodium | - | - | 1,2 g | 1,2 g |
| Monoéthanolamine ou Acide citrique | qsp pH 9 | qsp pH 9 | qsp pH 9 | qsp pH 9 |
| Eau déminéralisée | qsp 100g | qsp 100g | qsp 100g | qsp 100g |

La composition **B**' est ensuite appliquée sur les cheveux avec un rapport de bain de 4 g pour 1g de mèche ; le temps de pose est de 10 minutes à température ambiante. Au bout de quelques minutes une coloration très intense apparaît.

**Résultats colorimétriques :**

Après un rinçage, un shampooing et un séchage au casque des mèches, la coloration des cheveux est évaluée visuellement et lue au spectrocolorimètre Minolta CM3600d, illuminant D65, angle 10°, composante spéculaire incluse) pour les mesures colorimétriques L*, a*, b*.

**Chromaticité : C***

La chromaticité dans le système CIE L*, a*, b* est calculée selon l'équation suivante :

$$C^* = \sqrt{a^{*2} + b^{*2}}$$

Plus la valeur de C* est élevée plus la coloration obtenue est chromatique

| **Exemples** (sur cheveux à 90% de **blancs naturels**) | **13** | **14** | ***15*** | ***16*** |
|---|---|---|---|---|
| Composition (A'i) Etape 1 | A'1 | A'2 | *A'3* | *A'4* |
| Etape intermédiaire | **Non rincé, essuyé** | | | |
| Composition (B'i) Etape 2 | B'1 | B'2 | *B'3* | *B'4* |
| Nuances sur cheveux | cuivré | très léger beige | *doré vert* | *très léger beige* |
| Chromaticité (C*) | 34,30 | 17,66 | *30,71* | *20,63* |
| Intensité (L*) | 50,69 | 58,65 | *46,87* | *58,54* |

| **Exemples** (sur cheveux à 90% de blancs **naturels**) | **17** | **18** | ***19*** | ***20*** |
|---|---|---|---|---|
| Composition (Ai) Etape 1 | A'1 | A'2 | *A'3* | *A'4* |
| Etape intermédiaire | **Rincé, essuyé** | | | |
| Composition (Bi) Etape 2 | B'1 | B'2 | *B'3* | *B'4* |
| Nuances sur cheveux | doré vert | très léger beige | *doré vert* | *très léger beige* |
| Chromaticité (C*) | 30,18 | 16,97 | *33,39* | *19,50* |
| Intensité (L*) | 45,07 | 57,83 | *45,82* | *62,04* |

Il apparaît des tableaux ci-dessus que les mèches de cheveux blancs naturels traités avec les compositions selon l'invention permettent de colorer de façon significativement plus chromatique que les compositions comparatives respectives (voir A'1+B'1 vs. A'2+B'2 et A'3 + B'3 vs. A'4 + B'4) que ce soit avec le procédé faisant intervenir une étape intermédiaire de rinçage ou non rinçage, et essuyage). Par ailleurs les compositions selon l'invention procurent aux cheveux une coloration beaucoup plus intense que celles obtenues avec les comparatifs (L* plus faible avec les compositions selon l'invention).

**Revendications**

**1.** Procédé de coloration des fibres kératiniques, dans lequel lesdites sont traitées par :

i) un ou plusieurs dérivé(s) d'orthodiphénol(s),
ii) un ou plusieurs sel(s) métallique(s),
iii) du peroxyde d'hydrogène ou un ou plusieurs système(s) générateur(s) de peroxyde d'hydrogène ; et
iv) un ou plusieurs (bi)carbonate(s) ;

étant entendu que lorsque le procédé met en oeuvre un ou plusieurs système(s) générateur(s) de peroxyde d'hydrogène ledit procédé est réalisé en plusieurs étapes et le ou les (bi)carbonate(s) ne peuvent pas se retrouver appliqué(s) ensemble(s) sur les fibres avec le ou les sel(s) métallique(s).

**2.** Procédé de coloration selon la revendication précédente dans lequel le ou les orthodiphénol(s) choisis parmi les dérivé(s) d'orthodiphénol(s) naturel(s).

**3.** Procédé de coloration selon la revendication 1 ou 2 dans lequel l'ingrédient i) est un orthodiphénol à cycle aromatique choisi parmi le benzène, le naphtalène, le tétrahydronaphtalène, l'indane, l'indène, l'anthracène, le phénanthrène, l'isoindole, l'indoline, l'isoindoline, le benzofuranne, le dihydrobenzofuranne, le chromane, l'isochromane, le chromène, l'isochromène, la quinoléine, la tétrahydroquinoléine et l'isoquinoléine, ledit cycle aromatiques comportant au moins deux groupes hydroxy portés par deux atomes adjacents contigus du cycle aromatique.

**4.** Procédé de coloration selon une quelconque des revendications précédentes dans lequel l'ingrédient i) est de formule (I) suivante ou l'un de ses oligomères, sous forme salifié ou non :

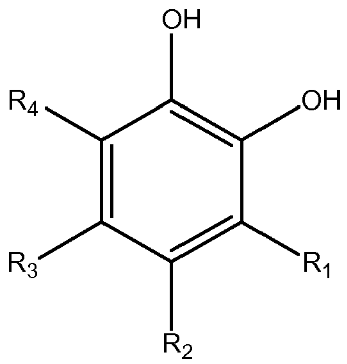

(I)

formule (I) dans laquelle les substituants :

- $R_1$ à $R_4$, identiques ou différents, représentent :

  - un atome d'hydrogène,
  - un atome halogène,
  - un radical hydroxy,
  - un radical carboxyle,
  - un radical carboxylate d'alkyle ou alcoxycarbonyle,
  - un radical amino éventuellement substitué,
  - un radical alkyle linéaire ou ramifié éventuellement substitué,
  - un radical alcényle linéaire ou ramifié éventuellement substitué,
  - un radical cycloalkyle éventuellement substitué,
  - un radical alcoxy,
  - un radical alcoxyalkyle,
  - un radical alcoxyaryle, le groupe aryle pouvant être éventuellement substitué,
  - un radical aryle,
  - un radical aryle substitué,
  - un radical hétérocyclique, saturé ou non, porteur ou non d'une charge cationique ou anionique, éventuellement substitué et/ou éventuellement condensé avec un cycle aromatique, ledit cycle aromatique étant éventuellement substitué,
  - un radical contenant un ou plusieurs atomes de silicium,

où deux des substituants portés par deux atome de carbone adjacents $R_1$ - $R_2$, $R_2$- $R_3$ ou $R_3$- $R_4$ forment conjointement un cycle saturé ou insaturé, aromatique ou non, substitué ou non, contenant éventuellement un ou plusieurs hétéroatomes et éventuellement condensé avec un ou plusieurs cycles saturés ou insaturés, éventuellement substitué, contenant éventuellement un ou plusieurs hétéroatomes.

**5.** Procédé de coloration la revendication précédente dans lequel le ou les orthodiphénol(s) sont choisis parmi :

- les flavonols,
- les anthocyanidines,
- les anthocyanines ou les anthocyanes ,
- les orthohydroxybenzoates,
- les flavones,
- les hydroxystilbènes,
- la 3,4-dihydroxyphénylalanine et ses dérivés,
- la 2,3-dihydroxyphénylalanine et ses dérivés,
- la 4,5-dihydroxyphénylalanine et ses dérivés,
- les dihydroxycinnamates,
- les orthopolyhydroxycoumarines,
- les orthopolyhydroxyisocoumarines,
- les orthopolyhydroxycoumarones,
- les orthopolyhydroxyisocoumarones,

- les orthopolyhydroxychalcones,
- les orthopolyhydroxychromones,
- les polyhydroxyquinones,
- les orthohydroxyxanthones,
- le 1,2 dihydroxybenzène et ses dérivés,
- le 1,2,4 trihydroxybenzène et ses dérivés,
- le 1,2,3 trihydroxybenzène et ses dérivés,
- le 2,4,5 trihydroxytoluène et ses dérivés,
- les proanthocyanidines,
- les proanthocyanines,
- l'acide tannique,
- l'acide ellagique,
- et les mélanges des composés précédents.

**6.** Procédé de coloration selon une quelconque des revendications 2 à 5 dans lequel le ou les orthodiphénol(s) naturel(s) i) sont choisis parmi les extraits d'animaux, de bactéries, de champignons, d'algues, de plantes.

**7.** Procédé de coloration selon la revendication précédente dans lequel le ou les orthodiphénol(s) naturel(s) i) sont choisis parmi :

- les extraits de feuilles de thé, les extraits de feuilles de romarin et les extraits de feuilles de maté ;
- les extraits de fruits, tels que les extraits de raisin, les extraits fèves et/ou cabosses de cacaoyer;
- les extraits de légumes, tels que les extraits de pelures d'oignon;
- les extraits de bois d'arbres, tels que les extraits d'écorce de pin, les extraits de bois de campêche.

**8.** Procédé de coloration selon une quelconque des revendications précédentes dans lequel le ou les sel(s) métallique(s) contenu(s) ii) sont choisis parmi les oxydes de Mn et Zn.

**9.** Procédé selon la revendication précédente dans lequel les sels de Mn et Zn, sont choisis parmi les halogénures, les sulfates, phosphates, nitrates et perchlorates, les sels d'acides carboxyliques tels que les gluconates ainsi que leurs mélanges.

**10.** Procédé de coloration selon une quelconque des revendications précédentes dans lequel la composition comprenant comme ingrédient iii) contient du peroxyde d'hydrogène ou du peroxyde d'urée.

**11.** Procédé de coloration selon une quelconque des revendications 1 à 9 dans lequel la composition comprenant comme ingrédient iii)

- les complexes polymériques pouvant libérer du peroxyde d'hydrogène choisi parmi le polyvinylpyrrolidone/$H_2O_2$ ;
- les oxydases produisant du peroxyde d'hydrogène en présence d'un substrat adéquat ;
- les perborates ; ou
- les percarborates.

**12.** Procédé de coloration selon une quelconque des revendications précédentes dans lequel l'ingrédient iv) contient du ou des (bi)carbonate(s) alcalins ou alcalino-terreux.

**13.** Procédé selon une quelconque des revendications précédentes en une étape consistant à appliquer sur les fibres kératiniques une composition aqueuse comprenant i), ii), iii), et iv) tels que définis dans une quelconque des revendications précédentes.

**14.** Procédé de coloration selon une quelconque des revendications 1 à 12 en deux étapes consistant dans la première étape à appliquer sur les fibres kératiniques une composition comprenant les ingrédients i), ii) et iii) tels que définis dans une quelconque des revendications 1 à 11, puis dans une deuxième étape consistant à appliquer une composition comprenant iv) tel que défini dans les revendications 1 ou 12, étant entendu qu'au moins une des deux compositions est aqueuse.

**15.** Procédé de coloration selon une quelconque des revendications 1 à 12 en deux étapes consistant dans la première

étape à appliquer sur les fibres kératiniques une composition comprenant les ingrédients i) et ii) tels que définis dans une quelconque des revendications 1 à 9 puis dans une deuxième étape consistant à appliquer une composition comprenant iii) et iv) tels que définis dans les revendications 1, 11 ou 12, étant entendu qu'au moins une des deux compositions est aqueuse.

**16.** Procédé de coloration selon une quelconque des revendications 1 à 15 réalisé en au moins deux étapes se terminant par le traitement des fibres kératiniques avec l'ingrédient iv) tel que défini dans les revendications 1 ou 12 et pouvant être suivi d'étapes de post- traitement tel que d'un shampooinage à l'aide de shampoing classique, d'un rinçage par exemple à l'eau et/ou du séchage des fibres kératiniques par traitement thermique ; étant entendu que ledit procédé ne fait pas intervenir de rinçage intermédiaire juste avant l'étape qui met en oeuvre l'ingrédient iv).

**17.** Procédé de coloration selon une quelconque des revendications 1 à 13, 15 et 16 réalisé en deux étapes dont la première étape est d'appliquer ensemble sur les fibres kératiniques les ingrédients i) et ii) tels que définis dans une quelconque des revendications 1 à 9 puis dans une deuxième étape d'appliquer ensemble les ingrédients iii) et iv) tels que définis dans une quelconque des revendications 1, 11 ou 12.

**18.** Procédé de coloration selon une quelconque des revendications 1 à 14 et 16 réalisé en deux étapes dont la première étape est d'appliquer ensemble sur les fibres kératiniques les ingrédients i), ii) et iii) tels que définis dans une quelconque des revendications 1 à 11 puis dans une deuxième étape d'appliquer le ou les ingrédients iv) tels que définis dans les revendications 1 ou 12.

**19.** Procédé de coloration selon une quelconque des revendications 1 à 18 dans lequel les fibres kératiniques sont juste avant l'étape qui met en oeuvre l'ingrédient iv) :

a) soit essuyées mécaniquement,
b) soit séchées par la chaleur avec un traitement thermique,
c) soit non rincées c'est-à-dire que les étapes se font successivement,

plus particulièrement juste avant l'étape qui met en oeuvre l'ingrédient iv) les fibres sont essuyées mécaniquement.

**20.** Composition cosmétique pour la coloration des fibres kératiniques contenant :

i) un ou plusieurs dérivé(s) d'orthodiphénol(s), tel(s) que défini(s) dans une quelconque des revendications 1 à 7 ;
ii) un ou plusieurs sel(s) métallique(s) tel(s) que défini(s) dans une quelconque des revendications 1, 8 et 9 ;
iii) du peroxyde d'hydrogène ou un ou plusieurs système(s) générateur(s) de peroxyde d'hydrogène tel(s) que défini(s) dans les revendications 10 ou 11 ; et
iv) un ou plusieurs (bi)carbonate(s) tels que définis dans une quelconque des revendications 1 ou 12.

**21.** Dispositif à plusieurs compartiments comprenant de 2 à 5 compartiments contenant de 2 à 5 compositions dans lesquels sont réparties les ingrédients i), ii), iii) et iv) tels que définis dans une quelconque des revendications 1 à 12, lesdites compositions étant aqueuses ou pulvérulentes, avec une au moins de ces compositions étant aqueuse.

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2814943
- FR 2814945
- FR 2814946
- FR 2814947
- US 5008093 A
- US 3376 A
- US 110 A
- US 5183901 A
- FR 2586913